# DEMANDE DE BREVET EUROPEEN

(11) **EP 0 722 697 A1**
(43) Date de publication de la demande: **24.07.1996**
(21) Numéro de dépôt: 96400084.8
(22) Date de dépôt: 12.01.1996
(51) Int. Cl.: A61B 17/70

(54) **Dispositif d'ostéosynthèse rachidienne à crochet médian et appuis d'ancrage vertébral**

(30) Priorité: 23.01.1995 FR 9500732
(71) Demandeur: SOCIETE DE FABRICATION DE MATERIEL ORTHOPEDIQUE SOFAMOR, F-93290 Tremblay-en-France (FR)
(72) Inventeur: Argenson, Claude, F-06000 Nice (FR); de Peretti, Ferdinand, F-06100 Nice (FR); Hovorka, Istvan, F-06200 Nice (FR)
(74) Mandataire: Martin, Jean-Paul

(57) **Abrégé**

Dispositif d'ostéosynthèse rachidienne comprenant, pour une même vertèbre, un crochet (12), deux moyens d'appui vertébraux associés audit crochet, lequel est adapté pour pouvoir prendre appui sur la partie médiane de l'arc postérieur vertébral entre deux tiges longitudinales (7) dudit dispositif, ce crochet étant équipé de moyens transversaux (28) de liaison entre le crochet et les tiges longitudinales avec possibilité de réglage dans un plan sagittal d'avant en arrière et de haut en bas et dans un plan frontal de gauche à droite entre les tiges et ledit crochet. Le crochet (12) et les deux vis ou crochets d'ancrage, assurent trois points d'appui sur la même vertèbre, ce qui évite d'avoir à instrumenter l'étage supérieur. De plus ce crochet peut être posé en fin d'intervention chirurgicale à l'étage désiré et à n'importe quel niveau choisi par le chirurgien, tout en permettant un gain de temps appréciable dans sa pose par rapport aux systèmes antérieurs.

## Description

La présente invention a pour objet un dispositif d'ostéosynthèse rachidienne du type comprenant deux tiges longitudinales s'étendant sur toute la longueur du segment vertébral à instrumenter et qui traversent les corps d'organes d'ancrage vertébral tels que des vis pédiculaires.

Ce dispositif est généralement équipé de systèmes de liaison transversale entre les tiges. En outre, on complète fréquemment cette instrumentation avec des crochets montés aux extrémités des tiges longitudinales et dont les parties terminales recourbées viennent prendre appui sur les côtés de l'arc postérieur de la vertèbre sus-jacente, en pénétrant par conséquent dans le canal médullaire de part et d'autre de la zone centrale de l'arc postérieur, et en appui sur une lame de cet arc.

Ces crochets terminaux accroissent la solidité et la rigidité de l'instrumentation. Cependant la pose de ces crochets nécessite d'instrumenter une vertèbre supplémentaire extérieure au segment rachidien concerné par le montage. De plus la durée de cette pose est relativement longue et augmente donc d'autant le temps de l'intervention chirurgicale.

L'invention a donc pour but de réaliser en même temps un système d'ancrage vertébral et de liaison transversale entre les deux tiges longitudinales du dispositif d'ostéosynthèse rachidienne, agencé de manière à éviter de prendre appui sur une vertèbre adjacente, non affectée par la pose de l'instrumentation, et qui puisse être mis en place en un temps réduit par rapport aux crochets utilisés jusqu'à présent.

Le dispositif d'ostéosynthèse rachidienne conforme à l'invention comprend, pour une même vertèbre, un crochet, deux moyens d'appui vertébraux associés audit crochet, lequel est adapté pour pouvoir prendre appui sur la partie médiane de l'arc postérieur vertébral entre deux tiges longitudinales dudit dispositif, ce crochet étant équipé de moyens transversaux de liaison entre le crochet et les tiges longitudinales avec possibilité de réglage dans un plan sagittal d'avant en arrière et de haut en bas et dans un plan frontal de gauche à droite entre les tiges et ledit crochet.

Les moyens d'appui vertébraux peuvent, notamment, être des vis ou des crochets pédiculaires.

Ainsi les deux crochets utilisés jusqu'à présent peuvent être remplacés par un seul crochet médian, sus-lamaire, de forme particulière adaptée à l'anatomie locale, prenant appui sur l'arc postérieur dans la partie la plus large du canal médullaire, et non sur les côtés de l'arc comme les crochets antérieurs.

Les moyens transversaux précités peuvent être des pattes fixées aux tiges longitudinales par tous éléments appropriés, par exemple des crochets standard de dispositifs de liaison transversale. Les extrémités de ces pattes peuvent être introduites dans des logements ménagés dans le corps du crochet. L'ensemble constitue ainsi un système d'ancrage vertébral et de liaison transversale entre les tiges longitudinales.

Un tel système présente l'avantage d'éviter d'instrumenter l'étage vertébral supérieur, en groupant sur le même étage trois points d'appui, avec possibilité de réglage du crochet médian par rapport aux deux autres appuis. Il peut de plus être posé par le chirurgien en un temps nettement plus court que la paire de crochets mise en oeuvre jusqu'à présent, par exemple d'environ une demi-heure.

Les pattes transversales, avantageusement coudées à angle droit permettent d'assurer l'ancrage du crochet en position sus-lamaire ou sous-lamaire (c'est-à-dire d'un coté ou de l'autre de la lame de l'arc vertébral), sur la même vertèbre que celle munie des vis ou crochets d'ancrage pédiculaire.

Suivant un mode de réalisation de l'invention, le crochet comporte une lame profilée et adaptée pour pouvoir s'appuyer sur une zone centrale de l'arc vertébral posté'rieur, dans la partie la plus large du canal médullaire du rachis, le corps comprenant deux parties latérales dans lesquelles sont formés lesdits alésages et un trou de passage de la vis correspondante, et ces deux parties latérales sont prolongées par des ergots s'étendant parallèlement à la lame et formant butées d'arrêt du crochet sur l'arc postérieur quant la lame est introduite dans le canal médullaire.

D'autres particularités et avantages de l'invention apparaîtront au cours de la description qui va suivre, faite en référence aux dessins annexés qui en illustrent une forme de réalisation à titre d'exemple non limitatif.

La figure 1 est une vue en élévation schématique d'une instrumentation d'ostéosynthèse rachidienne mise en place sur un segment du rachis pour la correction d'une déviation telle qu'une scoliose, une extrémité de cette instrumentation étant équipée du système d'ancrage vertébral et de liaison transversale selon l'invention.

La figure 2 est une vue en perspective partielle, à échelle agrandie, du système d'ancrage et de liaison transversale de la Fig.1 ainsi que de la vertèbre correspondante sur laquelle le crochet de ce système est ancré.

La figure 3 est une vue en élévation de la vertèbre de la Fig.2 suivant la flèche K montrant la zone d'appui de la lame du crochet du système de la Fig.2 ainsi que les points d'ancrage pédiculaire de l'instrumentation mise en oeuvre.

La figure 4 est une vue en élévation longitudinale schématique, à échelle agrandie, de la vertèbre des Fig.2 et 3 ainsi que du crochet médian du système d'ancrage vertébral associé.

La figure 5 est une vue en perspective à échelle ,agrandie du crochet des Fig.2 et 4.

La figure 6 est une vue en élévation latérale correspondant à la Fig.5.

La figure 7 est une vue de dessus du crochet de la Fig.5 et partielle de la vertèbre correspondante.

La figure 8 est une vue en perspective à échelle agrandie d'une patte coudée du système d'ancrage de la Fig.2.

La figure 9 est une vue en élévation schématique partielle analogue à la Fig.l représentant une variante d'exécution du système d'ancrage vertébral.

Les figures 10 et 11 sont des vues en élévation arrière, sensiblement à l'échelle, du système d'ancrage de la Fig.2, montrant ses possibilités de débattement du crochet en translation et en rotation dans un plan transversal perpendiculaire aux tiges longitudinales.

La figure 12 est une vue en élévation schématique latérale similaire à la Fig.4, à échelle réduite et montrant la position du crochet médian d'ancrage par rapport à l'épineuse d'une vertèbre thoracique adjacente à celle sur laquelle le crochet est posé.

La figure 13 est une vue en coupe partielle suivant 13 de la Fig.14 montrant trois points d'appui sur une vertèbre dont un constitué par la lame du crochet selon l'invention en position sous-lamaire.

La Fig.14 est une vue en élévation latérale avec arrachement de la vertèbre, du crochet sous-lamaire et de crochets pédiculaires.

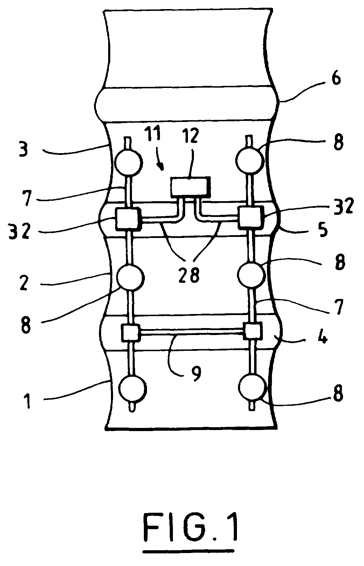

L'instrumentation d'ostéosynthèse schématiquement représentée à la Fig.1 s'étend sur un segment vertébral constitué de trois vertèbres 1, 2, 3, reliées par des disques vertébraux 4, 5, 6, et comprend deux tiges longitudinales 7, 8 ancrées dans les corps vertébraux à intervalles réguliers, de façon connue en soi au moyen de vis pédiculaires 8 schématiquement représentées.

L'instrumentation peut être complétée par un ou plusieurs dispositifs de liaison transversale (DLT), d'un type connu en soi, tel que le dispositif 9 reliant les tiges 7. Ces DLT peuvent être par exemple du genre décrit par les brevets français 2 645 427 du 11 avril 1989 et 2 659 225 du 8 mars 1990.

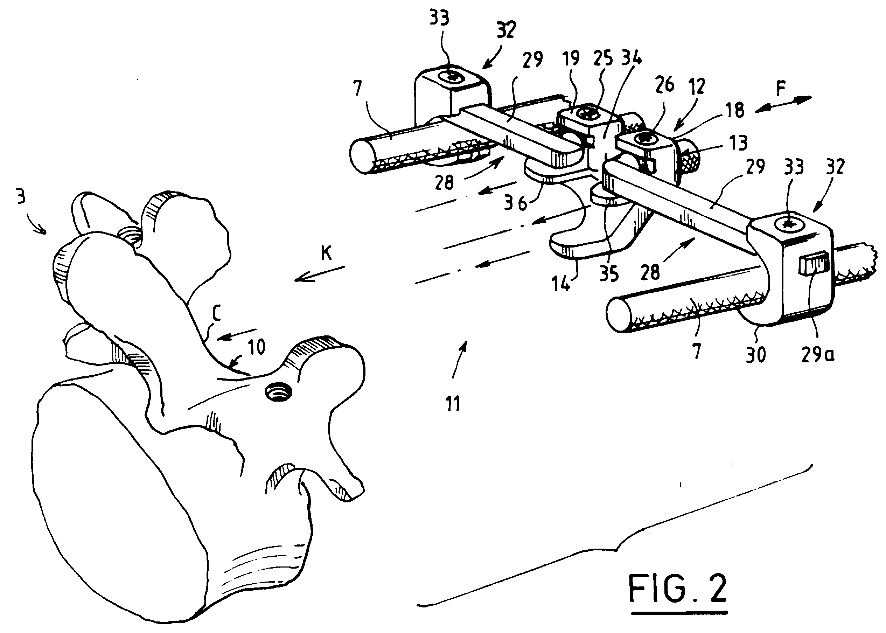

L'instrumentation est également équipée d'un système d'ancrage vertébral 11 et de liaison transversale entre les tiges 7, monté sur l'une des vertèbres d'extrémité du segment rachidien instrumenté, par exemple la vertèbre 3 dans l'exemple de la Fig.l. Ce système d'ancrage va être décrit en détail en se reportant plus particulièrement aux Fig.2 à 8.

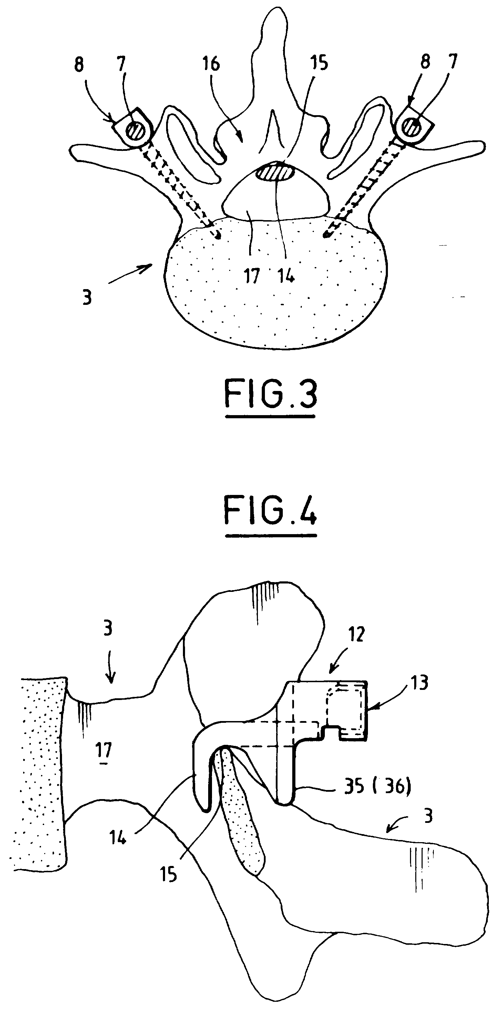

Il comprend tout d'abord un crochet médian 12 constitué d'un corps 13 et d'une lame médiane longitudinale 14 profilée et adaptée pour pouvoir s'appuyer sur une zone centrale 15 de l'arc vertébral postérieur 16, dans la partie la plus large du canal médullaire 17 du rachis (Fig.3). Le corps 13 est constitué de deux parties latérales 18, 19 dans lesquelles sont formés des alésages longitudinaux 21, 22, dont les axes longitudinaux sont parallèles au plan médian longitudinal P de la lame centrale 14 et qui sont symétriques de celui-ci. Dans ces alésages 21, 22 débouchent perpendiculairement des trous taraudés 23, 24 adaptés pour recevoir des vis 25, 26 respectives de blocage ou de fixation au corps 12 des extrémités cylindriques 20 de pattes coudées 28 de liaison du crochet 12 avec les tiges d'ostéosynthèse longitudinales 7.

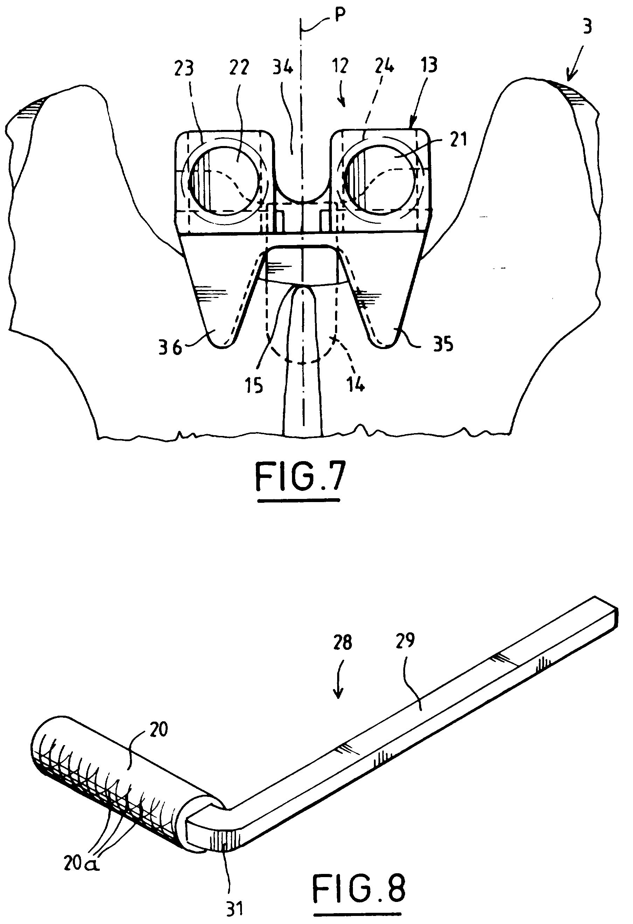

Chaque patte 28 est ainsi constituée d'une barrette transversale 29 à section par exemple rectangulaire (Fig.2 et 8) et à extrémité cylindrique 20 coudée à angle droit avec la barrette 29. Le diamètre de l'extrémité 20 lui permet de venir s'introduire dans l'alésage correspondant 21 ou 22. Ces parties cylindriques 20 sont reliées aux barrettes 29 par des zones de transition 31 dont la section est pratiquement constante afin de ne pas créer de zone de moindre résistance. La longueur des extrémités cylindriques 20, dont la surface est avantageusement à aspérités 20a (moletage, pointes de diamant...) est de préférence sensiblement supérieure à celle des alésages associés 21, 22 afin de permettre un réglage de la position longitudinale du crochet 12, dans une direction parallèle aux tiges 7, comme illustré par la double flèche F sur la Fig.2.

Les barrettes 29 peuvent être assujetties par leurs extrémités aux tiges longitudinales 7 par tout moyen approprié connu en soi, tel que des crochets 32 comportant un canal dans lequel vient se loger l'extrémité de la barrette correspondante 29. Une vis 33 vissée dans le corps du crochet 32 bloque la barrette 29 sur la tige 7 elle-même reçue dans la lame 30 du crochet 32.

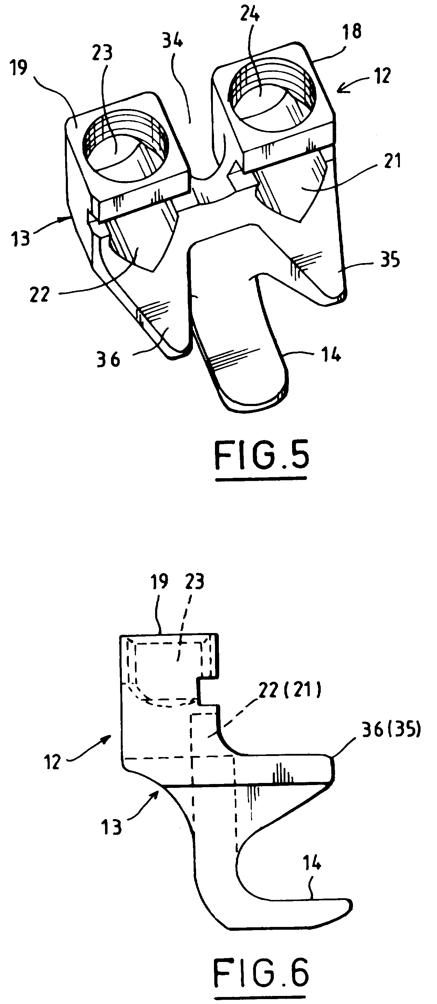

En se reportant en particulier aux Fig.5 et 6, on voit que les deux parties latérales 18, 19 du crochet médian 12 sont séparées par un canal ou évidement central longitudinal 34 et sont chacune prolongées par un ergot longitudinal 35, 36 s'étendant de chaque côté de la lame médiane 14 dans des directions longitudinales parallèles à celles de la lame 14.

Les ergots 35, 36 sont profilés pour pouvoir s'adapter à l'anatomie locale de l'arc postérieur de chaque côté de la zone centrale d'appui de la lame 14, afin de former pour celle-ci des butées d'arrêt empêchant une pénétration de la lame 14 dans le canal médullaire au-delà d'un point approprié (Fig.7).

La mise en place du système d'ancrage vertébral et de la liaison transversale qui vient d'être décrit se fait de la manière suivante : le chirurgien enfile tout d'abord dans les alésages 21, 22 les parties terminales cylindriques respectives 20 des pattes 28, en les plaçant dans la position longitudinale désirée, puis il effectue un prévissage des vis 25, 26 pour maintenir en place les extrémités 20. En second lieu les extrémités des barrettes 29 sont successivement introduites dans les canaux des crochets 32 jusqu'à ce que leurs extrémités 29a fassent légèrement saillie des crochets par des lumières de ceux-ci, puis le chirurgien exécute le vissage des vis 33 afin d'effectuer un blocage provisoire de l'ensemble du dispositif sur les tiges d'ostéosynthèse 7.

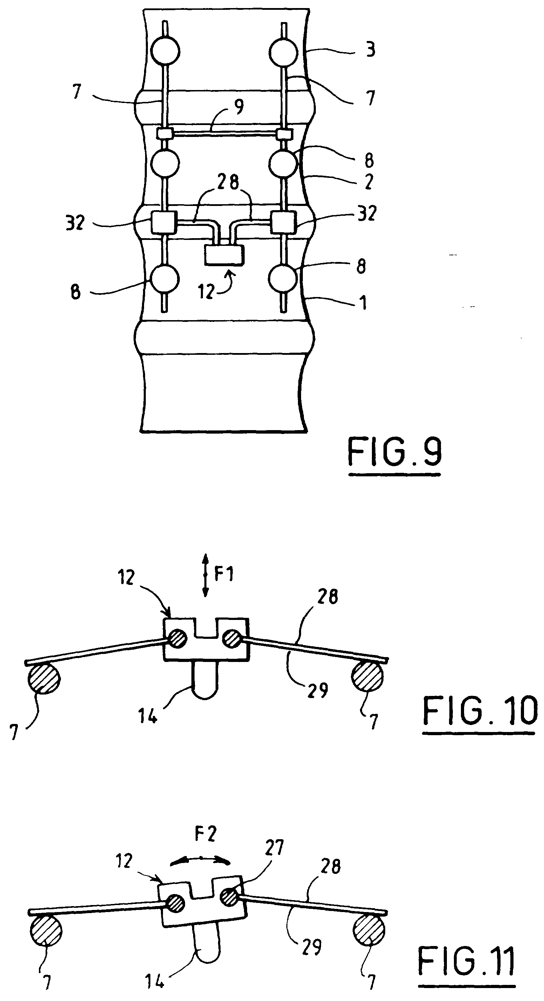

A ce stade, un réglage terminal peut être assuré dans deux directions, comme illustré aux Fig.10 et 11, c'est-à-dire dans un plan sagittal d'avant en arrière et de haut en bas, et dans un plan frontal de gauche à droite. A la Fig.10 on voit que, en raison du fait que le blocage des extrémités 20 par les vis 25, 26 n'est pas complet, il subsiste une possibilité d'articulation du crochet 12 sur ses extrémités 20 dans un plan transversal perpendiculaire au plan des tiges 7 (double flèche F1, Fig.10). De même le crochet médian 12 peut être basculé d'un côté ou de l'autre autour de l'une ou l'autre des extrémités 20 pour ajuster la position du crochet 12 à l'anatomie locale du patient, par rotation autour des extrémités cylindriques 20 (Fig.11, flèche F2).

Après positionnement définitif du crochet médian 12 dans le sens longitudinal (flèche F), dans le sens vertical (flèche F1, en supposant que le plan défini par les tiges 7 est horizontal), et enfin autour des parties ,cylindriques 20, les vis 25, 26 et 33 peuvent être définitivement bloquées pour maintenir fermement la lame 14 en appui contre la zone centrale 15 de l'arc postérieur 16 (Fig.2, 3 et 12). La pénétration dans le canal médullaire est arrêtée par les butées de sécurité 35, 36 venant en appui contre les côtés de l'arc postérieur 16 de part et d'autre de la zone centrale 15.

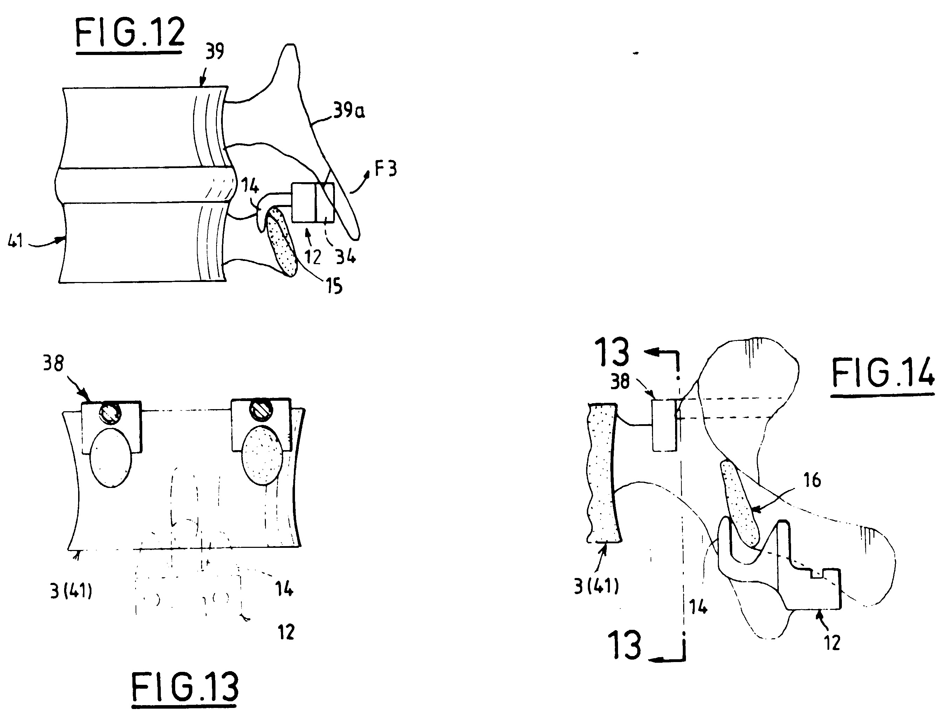

Cet appui par la lame 14 du crochet médian 12 peut être soit suslamaire (Fig.2 et 3), soit souslamaire comme représenté aux Fig.13 et 14. Dans les deux cas, le crochet 12 assure, avec les deux moyens d'ancrage pédiculaire sur la même vertèbre, soit par des vis pédiculaires 8 (Fig.3) soit par des crochets pédiculaires 38 (Fig.13 et 14), un système d'ancrage en trois points pour la vertèbre ainsi instrumentée, par exemple la vertèbre 3. Ainsi est réalisé un dispositif d'appui en trois points sur la même vertèbre, dans lequel le crochet médian 12 est placé en opposition avec les moyens d'appui pédiculaire pour stabiliser la vertèbre.

Pour certaines vertèbres, en particulier les vertèbres thoraciques 39 (Fig.12), l'évidement 34 fait fonction de canal de réception de l'épineuse 39a de ladite vertèbre une fois le crochet 12 mis en place sur la vertèbre contiguë 41. L'épineuse 39a est d'abord soulevée ou sectionnée par le chirurgien (flèche F3) pour faciliter la mise en place du crochet 12, après quoi l'épineuse est remise dans sa position initiale ou synthésée. L'épineuse 39a s'engage alors dans le canal longitudinal 34.

Dans la variante de réalisation représentée à la Fig.9, les pattes 28 et plus précisément leurs parties terminales 27 sont orientées de manière inverse de celles représentée à la Fig.l,c 'est-à-dire que ces extrémités 27 sont engagées dans les alésages 21, 28 par l'entrée de ces ,derniers opposée à celle des Fig.l et 2. Les barrettes 29 se retrouvent alors décalées longitudinalement de l'autre côté du corps 13, le résultat recherché étant par ailleurs évidemment le même. Il est également possible d'introduire l'une des extrémités 27 par une entrée de l'alésage 21 ou 22 opposée à celle par laquelle est introduite l'autre extrémité 27 dans l'alésage associé.

Dans les différentes formes de réalisation possible du crochet et du système d'ancrage vertébral incorporant ce crochet, l'invention présente les avantages suivants (outre les avantages déjà mentionnés) :
- Le système d'ancrage fait fonction en même temps de système de liaison transversale complémentaire à ceux tels que 9 déjà posés sur l'instrumentation d'ostéosynthèse, et renforce ainsi la rigidité de l'ensemble.
- L'invention permet de réaliser trois points d'appui sur la même vertèbre, à savoir l'appui par la lame 14 du crochet médian 12 et les deux appuis pédiculaires par les vis pédiculaires 8, ou les crochets pédiculaires 38. On évite ainsi d'instrumenter la vertèbre adjacente, non concernée par l'instrumentation d'ostéosynthèse, ce qui représente un avantage substantiel par rapport à l'état de la technique antérieure rappelé ci-dessus.
- La pose du crochet 12 selon l'invention avec ses pattes latérales 28 et ses moyens de fixation peut être assurée, comme déjà indiqué, en un temps nettement inférieur à celui nécessaire jusqu'à présent au chirurgien.
- Grâce aux articulations ménagées entre le crochet 12 et les pattes 28 par les extrémités cylindriques 20 et leurs alésages 21, 22, le crochet 12 peut être adapté à toutes les situations anatomiques particulières des barrettes 29 par rapport au rachis, car le crochet 12 peut monter ou descendre en faisant basculer les barrettes 29, et aussi se déplacer axialement pour rattraper des particularités anatomiques.
- Les ergots postérieurs 35, 36 constituent avantageusement des butées d'arrêt de la lame 14, qui empêchent celle-ci de pénétrer dans le canal médullaire 17 au-delà du point qui lui a été assigné, ce qui évite tout risque d'endommagement de la moelle épinière.
- Le crochet 12 et les pattes transversales 28 peuvent être mis en place à la fin de l'intervention chirurgicale, à l'étage vertébral désiré et à n'importe quel niveau, ce choix pouvant être fait en peropératoire par le chirurgien.
- Le temps de mise en place d'un DLT tel que 9 est pratiquement égal au temps de mise en place du crochet 12, ce qui correspond à un gain de temps d'environ 30 minutes par rapport au système antérieur à deux crochets plus un dispositif de liaison transversal. En effet il est important de souligner que le système selon l'invention fait également fonction de dispositif de liaison transversale, ce qui n'est pas le cas des crochets des dispositifs antérieurs connus. De ce fait l'invention permet, avec la pose d'un crochet unique, d'assurer à la fois un ancrage vertébral au niveau de l'arc postérieur et une liaison transversale, donc de supprimer la pose d'un dispositif DLT voisin.
- Le dispositif prévu par l'invention ne nécessite pas d'ancillaire spécifique, ceux des instrumentations existantes pouvant être utilisés sans difficulté.

Les indications du dispositif selon l'invention sont les suivantes :
a) Chaque fois que l'on veut terminer un montage par un crochet, c'est-à-dire en traumatologie, pour le traitement des scolioses et pour le traitement d'os porotiques.
b) Chaque fois que l'on veut compléter un montage par des vis et des crochets.

L'invention permet avantageusement de réaliser des pinces vertébrales, grâce aux trois appuis convenablement disposés comme indiqué ci-dessus, la lame 14 étant soit en position suslamaire, soit en position souslamaire (dans le second cas le crochet 12 est évidemment convenablement dimensionné et adapté). La pince ainsi obtenue offre la possibilité au chirurgien de réaliser diverses corrections et assure une meilleure tenue mécanique de la vertèbre instrumentée.

En variante, les alésages 21, 22 peuvent être réalisés différemment, en étant par exemple constitués par des logements sphériques recevant des sphères terminales des pattes 28.

## Revendications

1. Dispositif d'ostéosynthèse rachidienne comprenant deux tiges longitudinales (7) fixées sur le rachis et s'étendant respectivement de part et d'autre d'un tronçon de ce dernier, des moyens de liaison transversale s'étendant entre les deux tiges, au voisinage d'une vertèbre à laquelle chaque tige est reliée par un organe de fixation, caractérisé en ce qu'il comprend pour une même vertèbre, un crochet (12), deux moyens d'appui vertébraux (8, 38) associés audit crochet, lequel est adapté pour pouvoir prendre appui sur la partie médiane de l'arc postérieur vertébral (16) entre deux tiges longitudinales (7) dudit dispositif, ce crochet étant équipé de moyens transversaux (28) de liaison entre le crochet et les tiges longitudinales.

2. Dispositif selon la revendication 1, caractérisé en ce qu'il comprend des moyens de réglage de la position du crochet (12) par rapport aux tiges longitudinales (7), ces moyens de réglage étant adaptés pour autoriser un réglage de la position du crochet dans un plan sagittal, vers l'avant ou vers l'arrière et vers le haut ou vers le bas et dans un plan frontal vers la gauche ou vers la droite.

3. Dispositif selon la revendication 2, caractérisé en ce que le crochet (12) comprend un corps (13) dans lequel sont percés des logements (21, 22) de réception des extrémités des pattes transversales (28) de liaison entre le crochet et les tiges, et le crochet est équipé de moyens de fixation (25, 26) au corps des extrémités des pattes.

4. Dispositif selon la revendication 3, caractérisé en ce que lesdits logements sont constitués par des alésages longitudinaux (21, 22) et les pattes transversales (28) comportent des extrémités coudées (20) adaptées pour pouvoir s'engager dans les alésages précités.

5. Dispositif selon la revendication 4, caractérisé en ce que la longueur des extrémités coudées (20) est telle qu'elle permet un réglage de la position longitudinale du crochet (12) sur ces extrémités coudées.

6. Dispositif selon la revendication 3, caractérisé en ce qu'il comporte une lame (14) profilée et adaptée pour pouvoir s'appuyer sur une zone centrale (15) de l'arc vertébral postérieur (16), dans la partie la plus large du canal médullaire (17) du rachis, le corps comprenant deux parties latérales (18, 19) dans lesquelles sont formés lesdits alésages (21, 22) et des trous (23, 24) de passage des vis correspondantes (25, 26), et ces deux parties latérales sont prolongées par des ergots (35, 36) s'étendant parallèlement à la lame et formant butées d'arrêt du crochet (12) quand la lame est introduite dans le canal médullaire (17).

7. Dispositif selon la revendication 4, caractérisé en ce que les deux parties latérales (18, 19) du corps (13) sont séparées par un évidement longitudinal (34) de réception d'une épineuse (39a) d'une vertèbre adjacente (39).

8. Dispositif selon la revendication 5, caractérisé en ce que les pattes (28) sont constituées chacune d'une extrémité cylindrique (20) à aspérités (20a) de surface reçue dans un alésage correspondant (21, 22) et d'une barrette transversale (29) coudée à angle droit avec ladite extrémité cylindrique, et il est prévu un organe (32) de fixation de chaque patte à la tige d'ostéosynthèse (7) associée.

9. Dispositif selon la revendication 8, caractérisé en ce que les barrettes (29) sont à section rectangulaire et leur zone de transition (31) avec leur extrémité cylindrique (20) a une section pratiquement constante.
